# EUROPEAN PATENT APPLICATION

(11) **EP 3 797 784 A1**
(43) Date of publication of application: **31.03.2021**
(21) Application number: 19807885.9
(22) Date of filing: 22.05.2019
(51) Int. Cl.: A61K 33/00, A61P 7/02, A61P 9/04, A61P 9/08, A61P 9/10, A61P 17/14, A61P 43/00

(54) **ANTI-COAGULANT AGENT, BLOOD COAGULATION IMPROVING DEVICE, BLOOD COAGULATION IMPROVING METHOD, VASCULAR ENDOTHELIAL CELL FUNCTION IMPROVING METHOD, AND METABOLISM IMPROVING METHOD**

(30) Priority: 22.05.2018 JP 2018110698
(71) Applicant: H2bank Co., Ltd., Fukuoka-shi, Fukuoka 810-0001 (JP); Anicom Specialty Medical Institute Inc., Tokyo 160-0023 (JP)
(72) Inventor: ISHIBASHI Toru, Fukuoka-shi, Fukuoka 810-0001 (JP); ISHIHARA Genki, Tokyo 160-0023 (JP)
(74) Representative: Gill Jennings & Every LLP
(86) International application number: PCT/JP2019/020352
(87) International publication number: WO 2019/225669

(57) **Abstract**

An object of the present invention is to provide a method other than the scavenger function against reactive oxygen species for molecular hydrogen to improve life functions, in the fields of medical science, medical care, health industry, agriculture, animal husbandry, and fisheries, and an effect of improving mitochondrial function utilizing the same, and an anticoagulation agent, a blood coagulation curing device, a blood coagulation curing method, and a vascular endothelial cell function improving method. Provided is an anticoagulation agent composed of hydrogen gas or an anticoagulation agent composed of water containing molecular hydrogen. Preferably the anticoagulation agent is such that molecular hydrogen improves mitochondrial function by increasing mitochondrial hydrogenase activity, thereby improving blood clotting.

## Description

### TECHNICAL FIELD

The present invention relates to an anti-blood coagulation agent composed of hydrogen gas or a gas mixture containing hydrogen gas, or water containing molecular hydrogen, an anticoagulation device, a blood coagulation curing method, a vascular endothelial cell function improving method, and a metabolism improving method.

### BACKGROUND ART

Molecular hydrogen is known to have a scavenger function for reactive oxygen species, and the improvement effect of biological functions by hydrogen has also been considered to be mainly based on such a scavenger function.

A method of intaking molecular hydrogen by drinking a bio-adaptive liquid such as water which is added molecular hydrogen is a common method of ingesting gaseous molecular hydrogen, and examples of other methods of ingesting molecular hydrogen include a method of inhaling a gas containing molecular hydrogen, and a method such as infusion or transdermal absorption that enables direct electron transfer using a liquid containing molecular hydrogen.

The biological function-improving effects of hydrogen have received increasing attention in recent years and are becoming essential for human and pet health in particular. However, most of the life effects of hydrogen to date have been explained by its scavenger function, or the donation of electrons to reactive oxygen species that are extremely highly reactive, such as hydroxyl radicals or peroxynitrite (for example, Non Patent Documents 1 and 2). The above scope explains only part of life effects of molecular hydrogen, which in turn has limited applications of molecular hydrogen, or has undermined an important aspect of understanding life effects of molecular hydrogen.

### RELATED ART DOCUMENTS

### NON PATENT DOCUMENTS

Non Patent Document 1 Vascular Health and Risk Management, 2014, 10, 591-597
Non Patent Document 2 Current Pharmaceutical Design, 2013, 19, 6375-6381

### SUMMARY OF THE INVENTION

### PROBLEMS TO BE SOLVED BY THE INVENTION

An object of the present invention is to provide a method other than the scavenger function against reactive oxygen species for molecular hydrogen to improve biological functions, namely, improvement of mitochondrial function in the fields of medical science, medical care, health industry, agriculture, animal husbandry, and fisheries, and an anticoagulation agent, a blood coagulation curing device, a blood coagulation curing method, a vascular endothelial cell function improving method, and a metabolism improving method utilizing the same.

### MEANS FOR SOLVING THE PROBLEMS

In order to solve the above-described problems, the present inventors intensively studied to find that hydrogen has a function other than a scavenger function for reactive oxygen species, thereby completing the present invention.

Damage caused by stroke or myocardial infarction is life-threatening, and it is becoming clear that when ischemic mitochondria are supplied with oxygen through reperfusion, a burst of reactive oxygen species occurs, leading to an exacerbated prognosis. At the molecular level, it is thought that in the mitochondrial respiratory chain, electrons are normally transferred forward from complexes I to IV, whereas in the reverse electron transport (RET) that occurs during reperfusion after an ischemic state, for example, electrons flow back to complex I, resulting in explosive generation of ROS. The present inventors have found that hydrogen can inhibit such an event by acting on mitochondria, especially the electron transport chain thereof.

Although molecular hydrogen is very stable and the molecules do not dissociate under normal conditions in living organisms, the inventors have found that the dissociation of molecular hydrogen into electrons and protons, especially in mitochondrial complexes I and III, exerts a rectifying effect on the flow of electrons, which is the source of active oxygen by backflow or stagnation.

The rectifying effect of hydrogen on the flow of mitochondrial electrons was found to improve vascular endothelial cell function in a living organism as a result of inhibiting the generation of active oxygen itself.

Furthermore, the present inventors found that hydrogen can increase the mitochondrial hydrogenase activity and improve the blood coagulability when administered to a living organism, thereby completing the present invention.

Furthermore, the present inventors found that administration of hydrogen to a living organism can lead to mitochondrial uncoupling, resulting in a metabolic improvement effect, such as an increase in body temperature, thereby completing the present invention.

The present invention is as described in [1] to [10] below.
[1] An anticoagulation agent composed of hydrogen gas or a mixed gas containing hydrogen gas.
[2] An anticoagulation agent composed of water containing molecular hydrogen.
[3] The anticoagulation agent according to [1] or [2], wherein molecular hydrogen improves blood coagulation by increasing mitochondrial hydrogenase activity.
[4] An anticoagulation device characterized in that the device delivers hydrogen gas or a mixed gas containing hydrogen to a subject.
[5] The blood coagulation curing device according to [4], wherein molecular hydrogen improves blood coagulation by increasing mitochondrial hydrogenase activity.
[6] A blood coagulation curing method, including a step of administering water containing molecular hydrogen or hydrogen gas to a subject.
[7] A vascular endothelial cell function improving method, including a step of administering water containing molecular hydrogen or hydrogen gas to a subject.
[8] The vascular endothelial cell function improving method according to [7], wherein molecular hydrogen improves a vascular endothelial cell function by inhibiting generation of reactive oxygen species through a mitochondrial electron rectification.
[9] A method of protecting mitochondria, wherein molecular hydrogen improves a mitochondrial function or protects mitochondria by inhibiting generation of reactive oxygen species through mitochondrial electron rectification.
[10] A metabolism improving method, including a step of administering molecular hydrogen to a subject, wherein molecular hydrogen decreases a mitochondrial membrane potential in the subject by an uncoupling effect on mitochondria.

### EFFECTS OF THE INVENTION

Hydrogen is an electron-donating molecule that supported the energy of life in ancient times when there was no oxygen on earth, and hydrogenase, which has evolved primitive life by playing a role in hydrogen ion reduction and oxidation of molecular hydrogen, is evolutionarily linked to Complex I in the mitochondrial respiratory chain and has been a source of energy. The enhancement of mitochondrial hydrogenase activity by hydrogen is thought to be related to the above-described effect that dissociation of molecular hydrogen into electrons and protons in mitochondria exerts a rectifying effect on dysfunction of cellular respiration, which is the fundamental source of biological functions, and electron leakage, especially reverse electron transport (RET), in mitochondrial pathologies to contribute to health particularly in higher organisms, including humans.

The rectifying effect of molecular hydrogen on the mitochondrial electron transfer chain, enhancement of hydrogenase activity, and blood coagulation improvement, and the vascular endothelial cell function improving agents, blood coagulation improving agents, and devices using the same, are new effects and applications that cannot be explained by the function of molecular hydrogen as a scavenger of reactive oxygen species, which has been conventionally explained.

The present invention and a novel understanding of the background of the present invention can contribute to a clearer understanding of the biological effects of hydrogen ingestion and to new methods of hydrogen ingestion. More importantly, these findings will be useful in the development of drugs to improve the health of the electron transfer function of mitochondria, which is the fundamental source of energy metabolism in life and which has been difficult to control from outside the cell, and thus have immeasurable industrial benefits.

The present invention is extremely effective as a medical or health promotion method without side effects and provides fundamental information for future drug discovery, and its industrial effectiveness as well as health benefits is immeasurable.

### BRIEF DESCRIPTION OF THE DRAWINGS

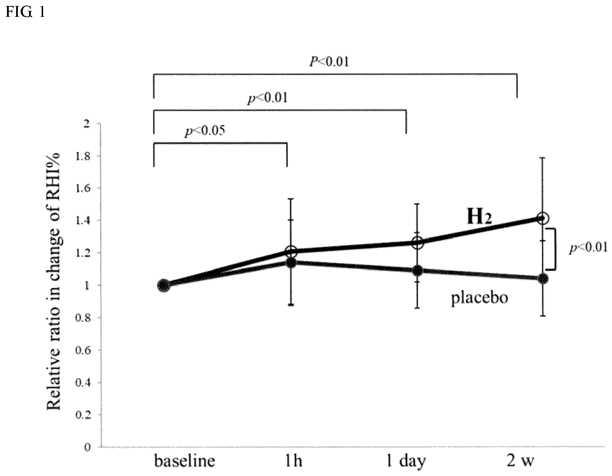

FIG 1 is a graph diagram illustrating the results of Example 8.

### MODE FOR CARRYING OUT THE INVENTION

An anticoagulation agent of the present invention is composed of gaseous molecular hydrogen (hydrogen gas) or a mixed gas containing the same, or water containing hydrogen gas.

In the case of hydrogen gas or a mixed gas containing hydrogen gas, the hydrogen gas can be administered to a subject (or animal) using a conventionally known hydrogen gas supply device. Specific examples of the administration method include inhalation of gas containing molecular hydrogen or transdermal absorption. The dosage of hydrogen gas per day per kilogram of living organism is preferably from 70 to 350 mg, and more preferably from 100 to 200 mg. In the case of a mixed gas, as long as the mixed gas is not harmful to a living organism, gases contained other than hydrogen are not particularly limited, and examples thereof include oxygen and air. In deep-sea diving, a mixed gas of hydrogen gas, helium gas, and oxygen gas has already been in practical use since the 1990s.

Water containing hydrogen gas can be administered to a living organism by providing it as drinking water as it is. When hydrogen gas is included in water, the content concentration is not particularly limited, and is preferably from 1.6 ppm to 8 ppm, and more preferably from 7 ppm to 8 ppm.

Water containing hydrogen gas may contain conventionally known components that are not toxic when administered to living organisms, such as salt and nutrients.

The anticoagulation (anti-blood coagulation) agent of the present invention can reduce von Willebrand factor activity. The von Willebrand factor (VWF) is a high-molecular-weight glycoprotein produced by vascular endothelial cells and bone marrow megakaryocytes, which forms a multimeric structure, and has a molecular weight ranging from about 500 kDa to as high as 20,000 kDa. VWF is a factor that plays an important role in primary hemostasis, and functions to adhere platelets to subendothelial connective tissue at a site of vascular injury. VWF exists as a multimer and varies in size, and the higher the molecular weight of the multimer, the greater the hemostatic capacity. VWF also has a function to stabilize FVIII in the plasma by binding to blood coagulation factor VIII (FVIII). Lowering the VWF factor activity means that blood is less likely to coagulate.

The anticoagulation agent of the present invention can increase the prothrombin time of a blood. Prothrombin is a factor II of a blood coagulation factor, and will clot when a substance called thromboplastin is added thereto. Accordingly, the prothrombin time is defined as the time it takes for plasma to clot when thromboplastin is added to the plasma. The anti-blood coagulation agent of the present invention can increase a partial thromboplastin time of blood. The partial thromboplastin time is a test that measures the blood coagulability of the endogenous system. The process of blood clotting involves a pathway known as the endogenous system and a pathway known as the exogenous system, each of which involves a number of blood coagulation factors.

The anticoagulation agent of the present invention is also expected to prevent occurrence of diseases such as embolism, thrombosis and atherosclerosis, such as cerebral and myocardial infarction, by inhibiting clotting of blood.

The anticoagulation agent of the present invention preferably improves blood clotting by increasing mitochondrial hydrogenase activity.

A blood coagulation curing device of the present invention supplies hydrogen gas or a mixed gas containing the same to a subject, and as long as hydrogen gas or a mixed gas containing the same can be supplied to a subject (human or animal), the configuration is not particularly limited, and a conventionally known medical hydrogen gas (hydrogen mixed gas) supply device can be used. Examples of the conventionally known medical hydrogen gas (hydrogen mixed gas) supply device include a hydrogen gas generator manufactured by Ecomo International, Inc.

A blood coagulation curing method of the present invention includes a step of administering water containing molecular hydrogen or hydrogen gas to a subject.

A vascular endothelial cell function improving method of the present invention includes a step of providing hydrogen to a subject (human or animal), wherein molecular hydrogen improves vascular endothelial cell function or protect mitochondria by inhibiting generation of reactive oxygen species through mitochondrial electron rectification. Improvements in vascular endothelial cell function can be evaluated by the Reactive Hyperemia Index (RHI). The RHI reflects the blood flow-responsive vasodilator function. In other words, molecular hydrogen has a mitochondrial electron rectifying effect, causing blood vessels to dilate and blood flow to increase.

Mitochondria are energy-converting devices that convert energy from food into the form of NADH or FADH₂ and, among other things, drive the ATP synthesizer (complex V) by passing electrons extracted from NADH to oxygen through an electron transfer chain containing complex I to be reduced to water and at the same time creating a concentration gradient of protons across a membrane.

The flow of electrons from NADH to oxygen is the forward electron transport (FET) in normal respiration. When cells become hypoxic (hypoxia) due to inadequate blood flow or ischemia, for example, succinic acid is elevated, and quinol (QH₂) reduced from quinone (Q10 in humans) in complex II flows back to complex I, and conversely, NAD+ is reduced to NADH in complex I (reverse electron transport (RET)). This electron flow results in electron leakage more than usual, and, for example, during reperfusion, when more oxygen is supplied, a leaked electron causes non-enzymatic reduction of oxygen by one electron, producing more superoxide, and as a result, generated hydrogen peroxide (H₂O₂) and hydroxyl radicals cause cellular and tissue damage.

In vascular endothelial cells, which are extremely important for vascular function, when there is an excess of the above-described reactive oxygen species, a normal vasodilating effect of nitric oxide (NO) is inhibited, and this is a major cause of endothelial cell dysfunction, which is a central factor in many lifestyle-related diseases.

In the present invention, for example, hydrogen (H₂) is used, but since hydrogen consists only of an electron and a proton, if hydrogen dissociates in the mitochondrial electron transfer chain and can supply protons and electrons, hydrogen provides a hitherto unknown flow of electrons in the electron transfer chain. In particular, complex I has evolved from hydrogenase, which used hydrogen as an electron source, and the structure at the active center of complex I is evolutionarily well preserved. In the following experiments, whether a pathological reactive oxygen species excess state can be improved by hydrogen changing the flow of electrons in response to the above-described RET or generation of superoxide in FET, which is a problem in ischemic conditions (or exerting a rectifying effect), was illustrated as Examples.

This means that a molecule that can appropriately donate both electrons and protons, like hydrogen, can contribute to health as an excellent rectifying molecule in the mitochondrial electron transfer chain.

### EXAMPLES

The present invention will be described in detail by way of Examples.

### [Example 1]

The von Willebrand factor activity, prothrombin time (PT) and partial thromboplastin time (PTT) of an adult who drank one bottle of 7 ppm hydrogen water (Ecomo International Inc.) per day (approximately 500 ml) for 4 weeks were measured after 4 weeks, according to a known method.

The results showed that after 4 weeks of drinking of hydrogen water, von Willebrand factor activity decreased by about 7%, prothrombin time (PT) was prolonged by about 4%, and partial thromboplastin time (PTT) was prolonged by about 5%. Before and after drinking, the von Willebrand factor activity, the prothrombin time (PT), and the partial thromboplastin time (PTT) of the adult male were all within normal ranges.

### [Example 2]

The effect of hydrogen on the oxidation of NADH or the reduction of NAD+ by mitochondria extracted from human cultured cells was examined. As a condition for observing RET, 1 mM NAD+ and 10 mM succinic acid were used as substrates and allowed to react in a buffer (50 mM phosphoric acid (pH 7.5), 1 mM EDTA, 0.2 mM Q10 (ubiquinone10), 0.1 mg/ml bovine serum albumin (BSA, without fatty acids)) for 25 min at 30°C, and the NADH concentration (average value per minute) was measured.

In the absence of hydrogen, 306 pmol/ml of NADH was detected, and in the presence of hydrogen (about 25 µM), the NADH concentration decreased to 165 pmol/ml, in other words, NADH was oxidized.

This means that hydrogen shifted the flow of electrons to the FET under RET conditions.

### [Example 3]

In addition to the above-described experimental conditions in Example 2, Strobilurin B, an inhibitor of complex III, was added to the experimental system to inhibit reactive oxygen species generated from complex III, and reactive oxygen species (in the presence of superoxide dismutase (SOD), superoxide was converted to H₂0₂, and measured as H₂0₂) from complex I was measured to be 16.4 µM in the absence of hydrogen, and decreased to 1.36 µM in the presence of hydrogen, a decrease of about one twelfth.

This means that under the conditions causing RET, hydrogen suppressed generation of reactive oxygen species and shifted the flow of electrons to the FET side.

### [Example 4]

In order to further observe generation of reactive oxygen species apart from electron flow, the same conditions as in Example 2 above were used, except that the complex I inhibitor, Q1, which is a water-soluble quinone, was used in place of the original electron carrier, Q10 (fat-soluble, moves in a lipid mitochondrial membrane, and functions as a carrier of electrons and protons in the electron transfer chain). As a result, H₂0₂ was increased from 10 µM in the absence of hydrogen to 195 µM in the presence of hydrogen. Contrary to the experiments in the presence of Q10 above, H₂O₂ increased by about 20 times.

This is thought to be because an electron donated by a dissociated hydrogen molecule reduced oxygen at the Qo site of complex III, which is a primary reactive oxygen species generating site, to generate a superoxide, and a water-soluble Q1 which is an electron acceptor but is completely reduced and cannot be transferred into a membrane becomes a semiquinone radical and generates a superoxide at the quinone binding site of complex I. This suggests that hydrogen is actually dissociated at the quinone-binding site in complex I or at the quinone-binding site (Qo) in complex III, and releases an electron. Conversely, the presence of a molecule such as Q10, which physiologically receives electrons in the mitochondria as an electron carrier, implies that hydrogen is an electron donor that does not generate reactive oxygen species.

### [Example 5]

Furthermore, the effect of hydrogen on the membrane potential of mitochondria was observed using a cell-penetrating cationic fluorescent dye Tetramethylrhodamine Ethyl Ester (TMRE), and the signal of TMRE, which represented the membrane potential, was reduced by about 10% (causing uncoupling) in the presence of molecular hydrogen. It is thought that the redox energy released from dissociated molecular hydrogen in Complex I or Complex III alters the concentration gradient of protons across the membrane.

The body temperature of an adult who drank two bottles (about 500 ml) of 7 ppm hydrogen water (Ecomo International Inc.) twice a day for about 6 months was measured after 6 months, and the basal body temperature was increased by about 0.5°C in the normal range. This was thought to be due to the above-described uncoupling effect of molecular hydrogen on adipose cell mitochondria.

This means an uncoupling effect of membrane potential, meaning that hydrogen has an effect similar to that of uncoupling protein and/or decoupling protein, which plays an important role in improving metabolism. Recently, it has been found that fat consumption by brown adipocytes and beige adipocytes, as well as the accompanying heat production and metabolic improvement, are important in, for example, preventing lifestyle-related diseases.

Conditions under which hydrogen causes uncoupling are influenced by the mitochondria membrane potential at the time of action of hydrogen when mitochondrial hydrogenase activity is activated, and for example, changes in the redox potential of the final electron acceptor cluster, the N2 cluster, in the array of Fe-S clusters transferring electrons from the NADH in complex I influenced by the electrochemical potential due to the proton gradient (or membrane potential) are considered to influence the above-described conditions. The relationship between the membrane potential at the time of action and the redox potential of the N2 cluster in complex I or the redox potential of the cytochrome C and Rieske Fe-S clusters, such as hame b_{L} and haem b_{H}, in complex III is considered to be important as a mechanism for hydrogen to induce uncoupling.

### [Example 6]

In order to achieve more physiological conditions, as a condition used for observing RET, in the experiment system of the above-described Example 2, in addition to 1 mM NAD+ and 10 mM succinic acid, 5 µM NADH was further added (NADH / NAD+ ratio is usually less than 1/100 in cells) as a substrate and allowed to react in a buffer (50 mM phosphoric acid (pH 7.5), 1 mM EDTA, 0.2 mM Q10 (ubiquinone10), 0.1 mg/ml bovine serum albumin (BSA, without fatty acids)) for 25 min at 30°C, and the NADH concentration (average value per minute) was measured. In the absence of hydrogen, 282 pmol/ml of NADH was detected, and in the presence of hydrogen (about 25 µM), the NADH concentration increased to 1,118 pmol/ml, in other words, NAD+ was reduced to NADH.

This means that even under RET conditions, hydrogen further shifted the electron flow to RET, depending on the concentration of NADH.

### [Example 7]

In the above-described experiment of Example 6, reactive oxygen species from complex I (in the presence of superoxide dismutase (SOD), superoxide was converted to H₂0₂, and measured as H₂0₂) was measured in Strobilurin B, an inhibitor of complex III, with reactive oxygen species generated from complex III suppressed, and the value was 23 µM in the absence of hydrogen, and decreased to 3.9 µM in the presence of hydrogen, which is about one fifth of the value in the absence of hydrogen.

This means that even in the presence of NADH and with increasing RET, hydrogen suppressed the generation of reactive oxygen species, and further shifted the electron flow toward RET depending on the concentration of NADH. Compared with the results in Example 2, which were obtained under conditions without NADH, the directions were shifted from the FET to the RET, and since hydrogen adjusts the direction of electron flow according to the ratio of NADH and inhibits the generation of reactive oxygen species under both conditions, it is thought to imply that hydrogen has an electron rectifying effect which suppresses electron leakage and transforms the direction of electron flow.

### [Example 8]

Improvement in RHI was measured in 32 patients with poor vascular endothelial function with an RHI value of less than 2 (RHI < 2, 15 in the placebo group, and 17 in the 7 pppm hydrogen drinking group). The 17 patients in the 7pppm hydrogen drinking group drank approximately 500ml of water containing 7ppm hydrogen per day, while the placebo group drank approximately 500ml of pure water per day, and the RHI was measured before drinking (first time), 1h after the start of drinking, 24h after the start of drinking (total of 2 doses), and at the end of 2 weeks. The RHI was measured using a Nihon Kohden Endopat 2000 (manufactured by Itamar Medical Ltd.) according to the specifications. From the initial measurement (baseline), the data at 1h, 24h, and 2 weeks are shown in Fig. 1.

The results showed that 7 ppm hydrogen water significantly improved the RHI of 32 patients with poor vascular endothelial function (RHI value < 2) after 2 weeks of continuous intake. As the effect of drinking of 7 ppm hydrogen water for peripheral vascular endothelial cells, the potential to improve vascular endothelial function in the poor RHI group, or a group at relatively high risk for cardiovascular disease was shown.

Vascular endothelial cells, which line blood vessels, are actually the largest endocrine organ in the human body, weighing about 1.5 kg in total. In order to eradicate lifestyle-related diseases, it is first and foremost necessary to ensure healthy functioning of vascular endothelial cells.

The reactive hyperemia index (RHI), obtained from the non-invasive test measuring fingertip 3D pulse amplitude tonometry, reflects endothelial cell function in peripheral blood vessels. The RHI is increasingly recognized as a factor that can predict human life prognosis independent of risk factors for lifestyle diseases such as metabolic syndrome, and is used by medical institutions worldwide as a powerful health screening tool.

It has been shown that individuals with low RHI are at an extremely high risk for future cardiovascular disease. With the exception of drugs, exercise and dietary control are the only ways to improve vascular function in such a population. It is important to clarify the role of hydrogen in low RHI populations at high risk for cardiovascular disease as a side effect-free, non-invasive improvement method.

### [Example 9]

The respiratory activity of mitochondria was measured in a mitochondrial respiratory chain, where reduction of oxygen to water was inhibited in the presence of a cyanide compound, by mixing molecular hydrogen.

### [Example 10]

The respiratory activity of mitochondrial complex III was measured in a mitochondrial respiratory chain, where reduction of oxygen to water was inhibited in the presence of a cyanide compound, by mixing molecular hydrogen.

## Claims

1. An anticoagulation agent composed of hydrogen gas or a mixed gas containing hydrogen gas.

2. An anticoagulation agent composed of water containing molecular hydrogen.

3. The anticoagulation agent according to claim 1 or 2, wherein molecular hydrogen cures blood coagulation by increasing mitochondrial hydrogenase activity.

4. An anticoagulation device **characterized in that** the device delivers hydrogen gas or a mixed gas containing hydrogen to a subject.

5. The anticoagulation device according to claim 4, wherein molecular hydrogen cures blood coagulation by increasing mitochondrial hydrogenase activity.

6. A blood coagulation curing method, including a step of administering water containing molecular hydrogen or hydrogen gas to a subject.

7. A vascular endothelial cell function improving method, comprising a step of administering water containing molecular hydrogen or hydrogen gas to a subject.

8. The vascular endothelial cell function improving method according to claim 7, wherein molecular hydrogen improves a vascular endothelial cell function by inhibiting generation of reactive oxygen species through a mitochondrial electron rectification.

9. A method of protecting mitochondria, wherein molecular hydrogen improves a mitochondrial function or protects mitochondria by inhibiting generation of reactive oxygen species through mitochondrial electron rectification.

10. A metabolism improving method, comprising a step of administering molecular hydrogen to a subject, wherein the molecular hydrogen decreases a mitochondrial membrane potential in the subject by an uncoupling effect on mitochondria.
